# EUROPEAN PATENT APPLICATION

(11) **EP 4 512 323 A1**
(43) Date of publication of application: **26.02.2025**
(21) Application number: 22938522.4
(22) Date of filing: 21.04.2022
(51) Int. Cl.: A61B 5/055

(54) **INFORMATION PROCESSING DEVICE, INFORMATION PROCESSING METHOD, AND RECORDING MEDIUM**

(71) Applicant: Cardio Flow Design Inc., Tokyo 102-0082 (JP)
(72) Inventor: NISHINO, Teruyasu, Tokyo 102-0082 (JP); ITATANI, Keiichi, Tokyo 102-0082 (JP)
(74) Representative: Plasseraud IP
(86) International application number: PCT/JP2022/018439
(87) International publication number: WO 2023/203722

(57) **Abstract**

The blood flow distribution determination unit 111 analyzes a first three-dimensional time-series image including an analysis target site of a subject imaged using phase-contrast imaging by an MRI (Magnetic Resonance Imaging) device to identify the blood flow velocity distribution of the analysis target site. The shape determination unit 112 analyzes a second three-dimensional time-series image indicating the anatomy of the subject imaged using a method different from phase-contrast imaging to identify the shape of the analysis target site. The image generation unit 113 generates a fused image by overlaying the first three-dimensional time-series image and the second three-dimensional time-series image. The index generation unit 114 generates an index related to blood flow flowing inside the shape based on the blood flow velocity distribution and the shape in the fused image. The output unit 115 associates the shape with the index and outputs them. This reduces the burden on subjects in blood flow measurements.

## Description

### Technical Field

The present invention relates to an information processing device, an information processing method, and a recording medium, specifically, to technology for generating indicators related to blood flow from MRI (Magnetic Resonance Imaging) images.

### Background Art

Techniques are known for visualizing the distribution of blood flow within blood vessels as vectors from images obtained by ultrasound diagnostic devices or MRI phase images (for example, see Patent Document 1).

### Prior Art Documents

### Patent Documents

Patent Document 1: International Publication No. 2013/077013

### Non-Patent Documents

Non-Patent Document 1: Itatani K, Sekine T, Yamagishi M, Maeda Y, Higashitani N, Miyazaki S, Matsuda J, Takehara Y. Hemodynamic Parameters for Cardiovascular System in 4D Flow MRI: Mathematical Definition and Clinical Applications. Magn Reson Med Sci. 2022; 21 (2) 380-399.

### Problem to be Solved by the Invention

Ultrasound has limitations in imaging certain areas, such as the distal ascending aorta and aortic arch, making it difficult to capture images of these regions. While MRI images can visualize these areas, contrast agents are often used to enhance image contrast for more accurate measurements. However, the use of contrast agents can be burdensome for subjects, and it is desirable to reduce the burden on subjects.

The present invention has been made in view of these points and aims to provide technology that reduces the burden on subjects during blood flow measurement.

### Means for Solving the Problem

The first aspect of the present invention is an information processing device. This device includes a blood flow distribution specifying unit that analyzes a first three-dimensional time-series image, which includes an analysis target region of a subject imaged by an MRI imaging device using a phase contrast method, to specify the blood flow velocity distribution of the analysis target region; a shape specifying unit that analyzes a second three-dimensional time-series image, which shows the anatomy of the subject and is imaged by a different method from the phase contrast method, to specify the shape of the analysis target region; an image generation unit that generates a fused image by overlaying the first three-dimensional time-series image and the second three-dimensional time-series image; an index generation unit that generates an index related to the blood flow inside the shape based on the blood flow velocity distribution and the shape in the fused image; and an output unit that outputs the shape and the index in association with each other.

The information processing device, which is the first aspect of the present invention, can be restated as follows. Specifically, it is an information processing device that includes: a blood flow distribution specifying unit that analyzes a first three-dimensional time-series image, which includes an analysis target region of a subject imaged by an MRI imaging device using the phase contrast method, to specify the blood flow velocity distribution of the analysis target region; a shape specifying unit that analyzes a second three-dimensional time-series image, which shows the anatomy of the subject and is imaged by a different method from the phase contrast method, to specify the shape of the analysis target region; an image generation unit that generates a fused image by overlaying the first three-dimensional time-series image and the second three-dimensional time-series image; an index generation unit that generates an index related to the blood flow inside the shape based on the blood flow velocity distribution and the shape in the fused image; and an output unit that outputs the shape and the index in association with each other.

The analysis target region may be the heart and great vessels of the heart. The shape specifying unit may include: a region dividing unit that divides a reference image, which is one of the time-series images constituting the second three-dimensional time-series image, into multiple regions, including the heart region; a feature point extraction unit that extracts one or more feature points in the reference image; a tracking unit that tracks the transition of the feature points in the second three-dimensional time-series image starting from the feature points; and a region changing unit that temporally changes the shapes of the multiple regions based on the tracking results of the feature points.

In the information processing device, which is the first aspect of the present invention, the analysis target region may be the heart and great vessels of the heart. The shape specifying unit may include: a region dividing unit that divides a reference image, which is one of the time-series images constituting the second three-dimensional time-series image, into multiple regions, including the heart region; a feature point extraction unit that extracts one or more feature points in the reference image; a tracking unit that tracks the transition of the feature points in the second three-dimensional time-series image starting from the feature points; and a region changing unit that temporally changes the shapes of the multiple regions based on the tracking results of the feature points.

The index generation unit may generate an index related to the blood flow inside the heart region specified by the shape specifying unit.

In the information processing device, which is the first aspect of the present invention, the index generation unit may generate an index related to the blood flow inside the heart region specified by the shape specifying unit.

The shape specifying unit may further include a grid point management unit that generates a grid within the reference image and deforms the grid based on the tracking results of the feature points. The image generation unit may generate an anatomical image with at least the second three-dimensional time-series image overlaid with the deformed grid.

In the information processing device, which is the first aspect of the present invention, the shape specifying unit may further include a grid point management unit that generates a grid within the reference image and deforms the grid based on the tracking results of the feature points. The image generation unit may generate an anatomical image with at least the second three-dimensional time-series image overlaid with the deformed grid.

The information processing device may further include an image acquisition unit that acquires a set of images where the time difference between the imaging time of the first three-dimensional time-series image and the imaging time of the second three-dimensional time-series image is within a predetermined time.

In the information processing device, which is the first aspect of the present invention, the information processing device may further include an image acquisition unit that acquires a set of images where the time difference between the imaging time of the first three-dimensional time-series image and the imaging time of the second three-dimensional time-series image is within a predetermined time.

The image acquisition unit may acquire the non-contrast second three-dimensional time-series image, which is imaged without using a contrast agent.

The information processing device, which is the first aspect of the present invention, may further include the image acquisition unit that acquires the non-contrast second three-dimensional time-series image, which is imaged without using a contrast agent.

A second aspect of the present invention is an information processing system. This information processing system includes: a blood flow distribution specifying means that analyzes a first three-dimensional time-series image, which includes an analysis target region of a subject imaged by an MRI (Magnetic Resonance Imaging) imaging device using the phase contrast method, to specify the blood flow velocity distribution of the analysis target region; a shape-specifying means that analyzes a second three-dimensional time-series image, which shows the anatomy of the subject and is imaged by a different method from the phase contrast method, to specify the shape of the analysis target region; an image-generating means that generates a fused image by overlaying the first three-dimensional time-series image and the second three-dimensional time-series image; an index generator means that generates an index related to the blood flow inside the shape based on the blood flow velocity distribution and the shape in the fused image; and an output means that outputs the shape and the index in association with each other. It should be noted that the information processing system described here is different from the information processing system S described later in the embodiments of the invention.

The information processing system, which is the second aspect of the present invention, can be restated as follows. Specifically, it is an information processing system that includes: a blood flow distribution specifying means that analyzes a first three-dimensional time-series image, which includes an analysis target region of a subject imaged by an MRI (Magnetic Resonance Imaging) imaging device using the phase contrast method, to specify the blood flow velocity distribution of the analysis target region; a shape specifying means that analyzes a second three-dimensional time-series image, which shows the anatomy of the subject and is imaged by a different method from the phase contrast method, to specify the shape of the analysis target region; an image generating means that generates a fused image by overlaying the first three-dimensional time-series image and the second three-dimensional time-series image; an index generating means that generates an index related to the blood flow inside the shape based on the blood flow velocity distribution and the shape in the fused image; and an output means that outputs the shape and the index in association with each other. It should be noted that the information processing system described here is different from the information processing system S described later in the embodiments of the invention.

A third aspect of the present invention is an information processing method. In this method, a processor executes the steps of: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

The information processing method, which is the third aspect of the present invention, can be restated as follows. Specifically, it is a computer-executed method that includes the steps of: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

The information processing method, which is the third aspect of the present invention, can be further restated as follows. Specifically, it is a method implemented on a computer which, using a processor, includes the steps of: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

A fourth aspect of the present invention is a recording medium. This recording medium stores a program that causes a computer to execute functions for: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other. This is a computer-readable recording medium.

The recording medium, which is the fourth aspect of the present invention, can be restated as follows. Specifically, it is a computer-readable recording medium storing a program that, when executed by a computer, causes the computer to perform the following steps: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

The recording medium, which is the fourth aspect of the present invention, can be further restated as follows. Specifically, it is a non-transitory computer-readable storage medium storing a program for causing a computer to perform the following steps: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

The fifth aspect of the present invention is a program. This program enables a computer to perform the following functions: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

The fifth aspect of the present invention, stated differently, is as follows. Specifically, it is a program product executed by a computer, which causes the computer to perform the following steps: analyzing a first three-dimensional time-series image, which includes an analysis target region of a subject imaged by an MRI imaging device using the phase contrast method, to specify the blood flow velocity distribution of the analysis target region; analyzing a second three-dimensional time-series image, which shows the anatomy of the subject and is imaged by a different method from the phase contrast method, to specify the shape of the analysis target region; generating a fused image by overlaying the first three-dimensional time-series image and the second three-dimensional time-series image; generating an index related to the blood flow inside the shape based on the blood flow velocity distribution and the shape in the fused image; and outputting the shape and the index in association with each other.

To provide this program or update a part of the program, a computer-readable recording medium containing this program may be provided, or the program may be transmitted via a communication network.

Furthermore, any combination of the above components, or transformation of the expression of the present invention into methods, devices, systems, computer programs, data structures, recording media, etc., is also effective as an aspect of the present invention.

The information processing apparatus and information processing system, according to the present disclosure, may be considered diagnostic support devices and diagnostic support systems, or diagnostic assistance devices and diagnostic assistance systems, respectively, based on the presentation of indices related to blood flow. Similarly, the information processing method and program according to the present disclosure may be considered as diagnostic support methods and diagnostic support programs, or diagnostic assistance methods and diagnostic assistance programs, respectively, based on the presentation of indices related to blood flow.

### Effect of the Invention

According to the present invention, the burden on the subject in blood flow measurement can be reduced.

### Brief Description of the Drawings

[Figure 1] It is a diagram illustrating an overview of an information processing system according to an embodiment.
[Figure 2] It is a diagram illustrating an overall configuration example of an information processing apparatus according to an embodiment.
[Figure 3] It is a diagram schematically showing a functional configuration example of the information processing apparatus according to an embodiment.
[Figure 4] It is a diagram schematically showing a functional configuration example of a shape identification unit according to an embodiment.
[Figure 5] It is a diagram illustrating feature point tracking performed by a shape identification unit according to an embodiment.
[Figure 6] It is a diagram illustrating the deformation of a grid performed by a shape identification unit according to an embodiment.
[Figure 7] It is a flowchart illustrating information processing executed by the information processing apparatus according to an embodiment.

### Embodiments for Implementing the Invention

### <Overview of Embodiments>

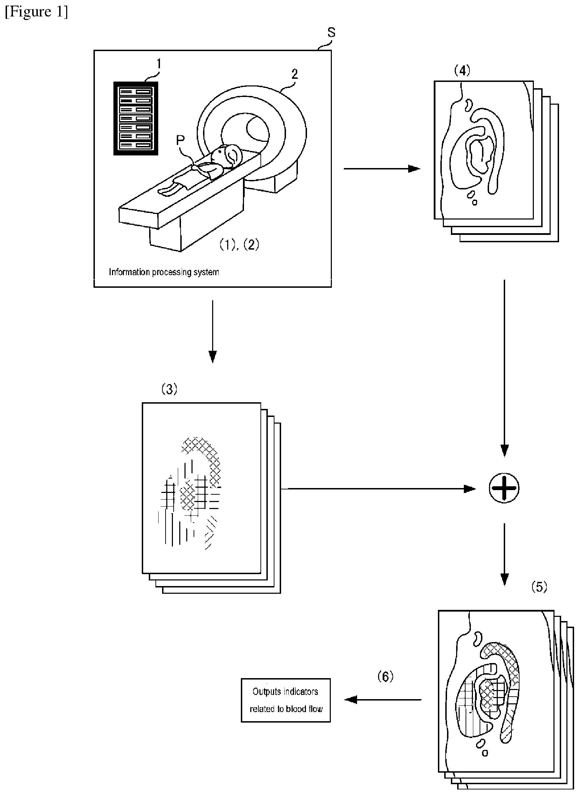

Figure 1 is a diagram illustrating an overview of an information processing system S according to an embodiment. Below, with reference to Figure 1, an overview of the embodiment is described.

The information processing system S, according to the embodiment, may include an information processing apparatus 1 and an MRI imaging device 2. In the information processing system S, according to the embodiment, medical practitioners such as doctors or radiographers may be able to acquire two different sets of images of the subject P in a single imaging sequence. One of the two different image sets may reflect the blood flow velocity distribution of the blood flowing through the analyzed region of the subject P, while the other may be images reflecting the anatomy of the subject P. The information processing apparatus 1 may aim to output indices related to the blood flow flowing through the body of the subject P by analyzing the two different image sets captured by the MRI imaging device 2.

In the example shown in Figure 1, medical practitioners may generate three-dimensional time series images of 4D Flow MRI and three-dimensional time series images called SSFP (Steady State Free Procession, or true FIESTA) using the MRI imaging device 2 as part of a single imaging sequence for the subject P as the subject p.

A detailed explanation is omitted as it is known in the art, but 4D Flow MRI is also referred to as the 3D cine phase-contrast method and is an MRI imaging method for imaging blood flow within the body of a subject P non-invasively. The phase-contrast method utilizes the fact that the precession motion of protons in three-dimensional time series data imaging of MRI is proportional to the velocity of water molecule movement by applying a gradient magnetic field, and it is an imaging method for obtaining the velocity distribution of blood flow in the direction of the gradient magnetic field. By slicing the velocity distribution of blood flow in the anterior-posterior, left-right, and superior-inferior directions and imaging analyzable regions such as the heart three-dimensionally, it is possible to visualize the three-dimensional blood flow within the analyzable region. This can be captured as cine images over the cardiac cycle; hence, it is called 4D Flow MRI. Therefore, the three-dimensional time series images of 4D Flow MRI reflect the distribution of blood flow velocity.

Furthermore, SSFP is a set of images that reflect the anatomy of the subject P. SSFP is commonly used in conventional cardiac MRI for measuring cardiac function. It should be noted that, in addition to SSFP, other imaging methods such as Fast Gradient Echo, Gradient Echo, or Black Blood methods can also be used to acquire image sets that reflect the anatomy of the subject P. All of these image sets can be captured by the MRI imaging device 2 and can be imaged together with the three-dimensional time series images of 4D Flow MRI in a single imaging sequence.

Below, the processing flow from imaging of the subject P to outputting indices related to blood flow in the information processing system S according to the embodiment is outlined in order (1) to (6), corresponding to (1) to (6) in Figure 1.

(1) Medical practitioners may generate first three-dimensional time series images reflecting the velocity distribution of blood flowing through the analyzable region of the subject P by imaging the subject P using the phase-contrast method.
(2) Medical practitioners may generate second three-dimensional time series images reflecting the anatomy of the subject P by imaging the subject P using a method different from the phase-contrast method in the same imaging sequence as the first three-dimensional time series images.
(3) The information processing device 1 may analyze the first three-dimensional time series images generated by the MRI imaging device 2 to identify the blood flow velocity distribution in the analyzable region. In the schematic diagram shown in Figure 1, regions indicated by different hatching patterns such as grids, diagonal lines, horizontal lines, and vertical lines may represent areas with different blood flow velocities. Since the first three-dimensional time series images represent the blood flow velocity distribution, the structure of the analyzable region may not necessarily be clearly imaged.
(4) The information processing device 1 may analyze the second three-dimensional time series images generated by the MRI imaging device 2 to identify the shape of the analyzable region. As schematically shown in Figure 1, the second three-dimensional time series image represents the anatomy of the analyzable region, and the boundaries of tissues may be imaged. However, information regarding blood flow velocity distribution within tissues may not be included.
(5) The information processing device 1 may generate a fused image by overlaying the first and second three-dimensional time series images. This allows the information processing device 1 to visualize the distribution of blood flow within the analyzable region.
(6) The information processing device 1 may generate indices related to blood flow within the shape of the analyzable region based on the blood flow velocity distribution and the shape of the analyzable region in the fused image (for example, refer to Itatani K, Sekine T, Yamagishi M, Maeda Y, Higashitani N, Miyazaki S, Matsuda J, Takehara Y. Hemodynamic Parameters for Cardiovascular System in 4D Flow MRI: Mathematical Definition and Clinical Applications. Magn Reson Med Sci. 2022; 21(2):380-399). As an example of "indices related to blood flow," these may include ventricular volume, ejection fraction, wall motion abnormalities, cardiac output, presence of accelerated blood flow, quantitative assessment of regurgitant flow at cardiac valves, quantitative assessment of intracardiac shunt ratio, quantitative assessment of local blood flow, flow passing through valves such as the aortic valve, mitral valve, pulmonary valve, tricuspid valve, wall shear stress (WSS) at vessel walls, indices of temporal variation of WSS, flow and energy loss in the aorta, deformation of blood vessels and heart (changes in longitudinal stretching and contraction, curvature of the aortic arch, torsion rate, changes in ventricular volume, ejection fraction), and so on.

This way, the information processing system S, according to the embodiment, may track the motion of analyzable regions without the use of contrast agents by combining image sets such as SSFP, which represent the anatomy of the subject P, with image sets of 4D Flow MRI, which represent blood flow velocity distribution. Additionally, since the information processing system S can capture two different image sets in a single imaging sequence, imaging time can be shortened. Consequently, the information processing system S can reduce the burden on the subject P during blood flow measurement.

### <Functional Configuration of the Information Processing Device 1 in the Embodiment>

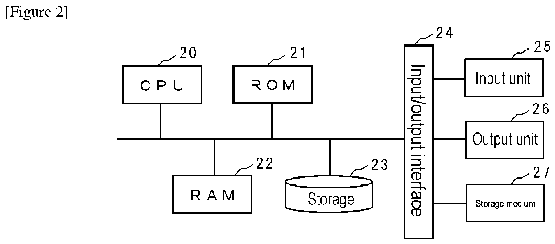

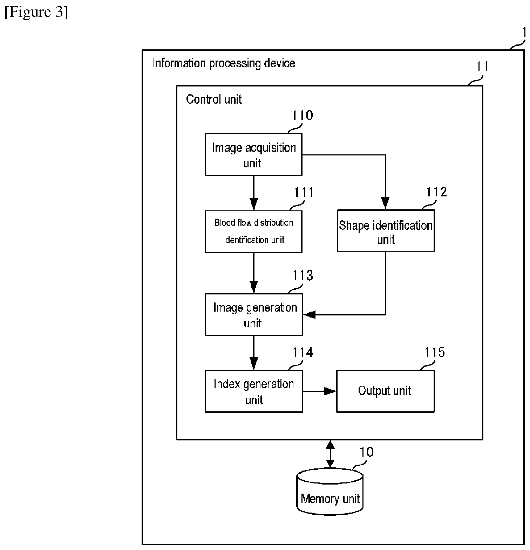

Figure 2 illustrates an example of the overall configuration of the information processing device 1. As shown in this figure, the overall configuration of the information processing device 1 may include a CPU 20 capable of performing arithmetic processing, ROM 21 capable of storing BIOS and the like, RAM 22 that may serve as a working area, and a storage 23 capable of storing programs, and so forth. Additionally, the overall configuration of the information processing device 1 may further include an input unit 25, an output unit 26, and a storage medium 27 via an input/output interface 24. The input unit 25 may include input devices such as keyboards, while the output unit 26 may include output devices such as displays. Data transmission between the MRI imaging device 2 and the information processing device 1 may be conducted via the input unit 25 and the output unit 26. The functional configuration of the information processing device 1 shown in Figure 3 may be included in the overall configuration shown in Figure 2. In the configuration of Figure 3, storage devices such as ROM 21, RAM 22, and storage 23 may be consolidated into a memory unit 10.

Figure 3 schematically illustrates an example of the functional configuration of the information processing device 1 in the embodiment. The information processing device 1 may be, for example, a workstation for medical use, equipped with a memory unit 10 and a control unit 11. In Figure 3, arrows indicate the main data flow, and there may be additional data flows not shown in the figure. In Figure 3, each functional block may represent a functional unit rather than a hardware (device) unit. Therefore, the functional blocks shown in Figure 3 may be implemented within a single device or divided into multiple devices. Data exchange between functional blocks may be performed via data buses, networks, portable storage media, or any other means.

The memory unit 10 may include ROM (Read Only Memory) storing BIOS (Basic Input Output System) of the computer realizing the information processing device 1, RAM (Random Access Memory) serving as the working area of the information processing device 1, and large-capacity storage devices such as HDD (Hard Disk Drive) or SSD (Solid State Drive) that store OS (Operating System), application programs, and various information referenced during the execution of said application programs.

The control unit 11 may be a processor such as the CPU (Central Processing Unit) or GPU (Graphics Processing Unit) of the information processing device 1. By executing programs stored in the memory unit 10, the control unit 11 may function as an image acquisition unit 110, blood flow distribution identification unit 111, shape identification unit 112, image generation unit 113, index generation unit 114, and output unit 115.

Note that Figure 3 illustrates an example where the information processing device 1 is configured as a single device. However, the information processing device 1 may be implemented using multiple calculation resources, such as processors and memory, for example, in a cloud computing system. In this case, each component constituting control unit 11 may be realized by at least one of the processors executing the program among multiple different processors.

The image acquisition unit 110 may acquire a first three-dimensional time-series image, including the analysis target area of the subject p imaged using phase-contrast imaging method by the MRI imaging device 2. Additionally, the image acquisition unit 110 may acquire a second three-dimensional time-series image showing the anatomy of the subject p. Here, the second three-dimensional time-series image may be image data acquired using a different method (e.g., SSFP) from imaging using phase-contrast imaging.

The blood flow distribution identification unit 111 may identify the blood flow velocity distribution of the analysis target area by analyzing the first three-dimensional time-series image using known blood flow visualization techniques. Furthermore, the blood flow distribution identification unit 111 may analyze the first three-dimensional time-series image, including the analysis target area of the subject imaged, using phase-contrast imaging by the MRI (Magnetic Resonance Imaging) imaging device to identify the blood flow velocity distribution of the analysis target area. The shape identification unit 112 may analyze the second three-dimensional time-series image to identify the shape of the target area. Additionally, the shape identification unit 112 may analyze the second three-dimensional time-series image showing the anatomy of the subject acquired using a method different from imaging using phase-contrast imaging to identify the shape of the target area.

The image generation unit 113 may generate a fused image by using known medical image synthesis techniques to overlay the first three-dimensional time-series image and the second three-dimensional time-series image. In other words, the image generation unit 113 may generate a fused image by overlaying the first three-dimensional time-series image and the second three-dimensional time-series image, thereby visualizing the relationship between the shape of the analysis target area and the blood flow velocity distribution.

The index generation unit 114 may generate indices related to the blood flow flowing within the shape based on the blood flow velocity distribution and the shape in the fused image. The index generation unit 114 may generate indices related to the blood flow flowing within the region of the heart identified by the shape identification unit 112. More specifically, the index generation unit 114 may generate indices related to the blood flow flowing within the region of the heart identified by the shape identification unit 112. From the relationship between the internal structure of the heart and the blood flow velocity within the heart, the index generation unit 114 may accurately calculate indices such as ventricular volume, ejection fraction, wall motion abnormalities, stroke volume, presence of accelerated blood flow, quantification of regurgitant volume in heart valves, quantification of intracardiac shunt rate, quantification of local blood flow volume, flow passing through valves such as the aortic valve, mitral valve, pulmonary valve, and tricuspid valve, wall shear stress (WSS) on the vascular wall, indices of temporal variation in WSS, flow and energy loss in the aorta, deformation of blood vessels and heart (changes in longitudinal stretching and compression, curvature of the aortic arch, torsion rate, changes in ventricular volume, ejection fraction), and the like. The output unit 115 may correspondingly associate the shape with the indices and output them to a display unit (not shown) of the information processing device 1 or to a terminal (not shown) communicable with the information processing device 1.

An information processing device (referred to as "information processing device 1") comprising a blood flow distribution identification unit that analyzes first three-dimensional time-series images including analysis target areas of subjects imaged using phase-contrast imaging by an MRI imaging device to identify blood flow velocity distribution of the analysis target areas; a shape identification unit that analyzes the second three-dimensional time-series images showing the anatomy of subjects imaged using a method different from phase-contrast imaging to identify the shape of the analysis target areas; an image generation unit that generates fused images by overlaying the first three-dimensional time-series images and the second three-dimensional time-series images; an index generation unit that generates indices related to the blood flow flowing within the shape based on the blood flow velocity distribution and the shape in the fused images; and an output unit that associates the shape with the indices and outputs them can reduce the burden on the subject in blood flow measurements.

Thus, as described above, the blood flow distribution identification unit can analyze the first three-dimensional time-series images to identify the blood flow velocity distribution for the analysis target areas of the subject, while the shape identification unit can be configured to analyze the second three-dimensional time-series images to identify the shape of the analysis target areas. Furthermore, the image generation unit may be configured to generate fused images by overlaying the first three-dimensional time-series images and the second three-dimensional time-series images. Additionally, the index generation unit may generate indices related to the blood flow flowing within the aforementioned shape, and the output unit may correspondingly associate the aforementioned shape with the aforementioned indices for output. Therefore, to obtain the output corresponding to the aforementioned shape and indices, it is sufficient to acquire the first three-dimensional time-series images and the aforementioned second three-dimensional time-series images, thereby reducing the burden on the subject, i.e., the examinee, without imposing significant stress.

### <Functional Configuration of the Shape Identification Unit 112>

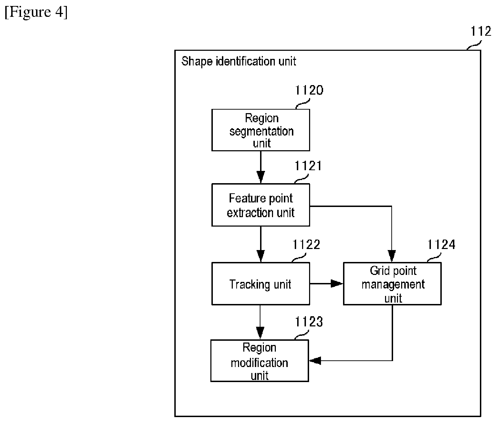

Figure 4, which explains this configuration, schematically illustrates an example of the functional configuration of the shape identification unit 112 according to an embodiment. The shape identification unit 112, according to an embodiment, may include a region segmentation unit 1120, a feature point extraction unit 1121, a tracking unit 1122, a region modification unit 1123, and a grid point management unit 1124. Below, assuming that the analysis target area targeted by the information processing device 1 according to an embodiment is the heart and major blood vessels of the subject P, the functional configuration of the shape identification unit 112 will be described. However, it should be understood by those skilled in the art that the target area of the analysis is not limited to the heart and major blood vessels but may include other areas where blood flows.

To improve the accuracy of diagnosing circulatory diseases such as heart conditions using 4D Flow MRI, cardiac cycle tracking is known to be effective. Conventionally, to more accurately track the pulsation of the heart and major blood vessels, contrast agents such as blood pool contrast agents have been administered to subject P to increase contrast during imaging. However, there is a desire to reduce the use of contrast agents and shorten imaging times from the perspective of reducing the burden on subject P who may have reduced cardiac function.

Therefore, in the embodiment, the shape identification unit 112 may execute pulse tracking using a tracking method based on, for example, the well-known Lucas-Kanade method, utilizing an image set indicating anatomy such as SSFP instead of 4D Flow MRI as the analysis target. Firstly, the region segmentation unit 1120 may divide a reference image, which is one of the multiple time-series images composing the second three-dimensional time-series images, into multiple regions, including the heart region.

The feature point extraction unit 1121 may extract one or more feature points in the reference image. The feature points extracted by the feature point extraction unit 1121 may include, for example, the edges of regions such as the heart and major blood vessels. The tracking unit 1122 may track the transition of feature points in the second three-dimensional time-series images, starting from the feature points extracted by the feature point extraction unit 1121. Consequently, the tracking unit 1122 may track the pulsation, which is the temporal movement of the analysis target area. The region modification unit 1123 may change the shapes of multiple regions based on the tracking results of the feature points and may further display them on a display unit.

In the aforementioned information processing device 1, where the analysis target area is the heart and major blood vessels, the shape identification unit 112 includes a region segmentation unit 1120 that divides a reference image, which is one of the multiple time-series images composing the second three-dimensional time-series images, into multiple regions including the heart region, a feature point extraction unit 1121 that extracts one or more feature points in the reference image, a tracking unit 1122 that tracks the transition of feature points in the second three-dimensional time-series images starting from the feature points, a region modification unit 1123 that changes the shapes of multiple regions based on the tracking results of the feature points, and a region modification unit 1124 that changes the shapes of multiple regions based on the tracking results of the feature points. Such an information processing device may effectively grasp the temporal variation of the analysis target area in blood flow measurement.

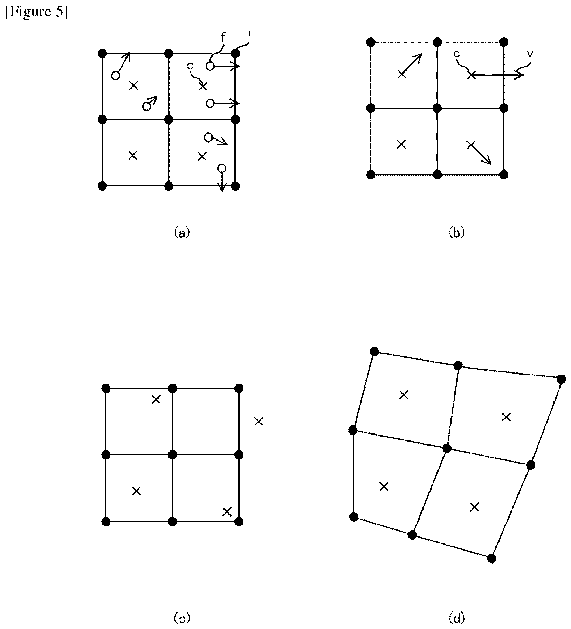

This is to act as follows, in conjunction with the explanation of Figures 5(a)-(d) to be described later. That is, the region segmentation unit 1120 divides a reference image, which is one of the multiple time-series images composing the second three-dimensional time-series images, into multiple regions including the heart region, the feature point extraction unit 1121 extracts one or more feature points in the reference image, the tracking unit 1122 tracks the transition of feature points in the second three-dimensional time-series images starting from the feature points, the region modification unit 1123 changes the shapes of multiple regions based on the tracking results of the feature points, and the region modification unit 1124 changes the shapes of multiple regions based on the tracking results of the feature points. After processing by the image generation unit 113 and the index generation unit 114, the output unit 115 may output the corresponding shapes and indices as described above.

Furthermore, by configuring the index generation unit 114 to generate indices concerning the blood flow flowing inside the region of the heart identified by the shape identification unit 112, the output unit may output the corresponding shapes and indices as described above, particularly generating indices regarding the blood flow inside the heart.

Figures 5(a)-(d) illustrate feature point tracking performed by the shape identification unit 112 according to the embodiment. In Figures 5(a)-(d), the black circles labeled with symbol 'l' represent grid points of a square grid set in the reference image. Not all grid points are labeled to avoid clutter, but similar black circles as those labeled with symbol 'l' in Figures 5(a)-(d) represent grid points. The grid consisting of grid points shown in Figure 5(a) is a grid generated by the grid point management unit 1124 of the shape identification unit 112 in the reference image.

As shown in Figure 5(a), the grid consists of square regions formed by four grid points 'l'. In Figures 5(a)-(d), the crosses labeled with symbol 'c' represent the centroids of the square regions formed by four grid points 'l'. Not all centroids are labeled to avoid clutter, but similar crosses, such as those labeled with symbol 'c' in Figures 5(a)-(d), represent the centroids of the square regions.

Furthermore, the white-outlined circles labeled with symbol 'f in Figures 5(a)-(d) represent the feature points extracted by the feature point extraction unit 1121. Not all feature points are labeled to avoid clutter, but similar white-outlined circles as those labeled with symbol 'f in Figures 5(a)-(d) indicate feature points.

In Figure 5(a), the arrows starting from the feature points 'f represent the movement vectors of the feature points 'f, indicating the tracking results of the feature points 'f by the tracking unit 1122. Figure 5(a) illustrates how the feature points 'f in the reference image moved in adjacent images (i.e., frames adjacent to the reference image) over time.

The region modification unit 1123 may calculate centroid displacement vectors, which are weighted averages based on distances from the centroids of the respective quadrilateral regions to the movement vectors of the feature points 'f contained in each quadrilateral region. In Figure 5(b), arrows starting from the centroids 'c' represent centroid displacement vectors. Not all centroid displacement vectors are labeled to avoid clutter, but similar arrows as those labeled with symbol 'v' in Figure 5(b) represent centroid displacement vectors. Thus, the region modification unit 1123 can represent the temporal variations of each quadrilateral region with vectors.

Figure 5(c) illustrates the relationship between the grid points 'l' in the reference image and the centroids 'c' of the quadrilateral regions after movement. The region modification unit 1123 may adjust the positions of the grid points 'l' so that the centroids 'c' of the quadrilateral regions after movement become the centroids 'c' of the quadrilateral regions. Figure 5(d) shows new grid points 'l' modified by the region modification unit 1123 based on the movement of the centroids 'c' of the quadrilateral regions. In the example shown in Figure 5(d), the quadrilateral regions formed by the four grid points 'l' are expanded, corresponding to the expansion due to the heartbeat of the heart.

Returning to the explanation of Figure 4, the grid point management unit 1124 may deform the grid generated in the reference image based on the tracking results of the feature points 'f by the tracking unit 1122.

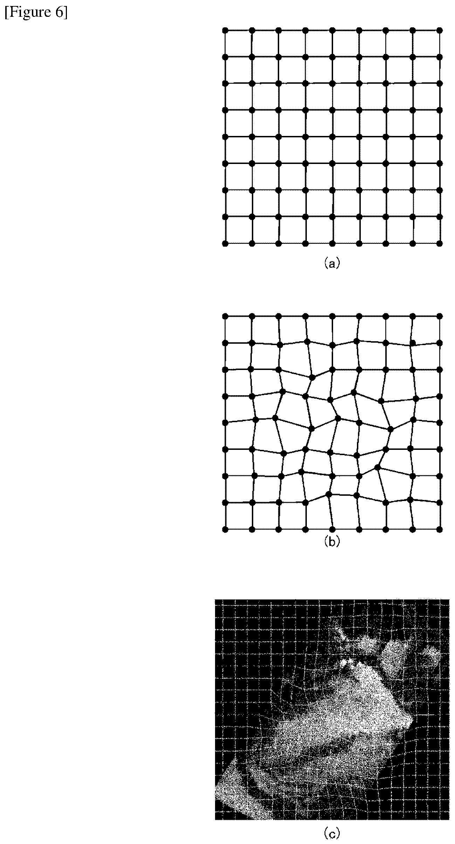

Figures 6(a)-(c) explain the deformation of the grid performed by the shape identification unit 112 according to the embodiment. Specifically, Figure 6(a) illustrates the grid in the reference image, Figure 6(b) shows the deformed grid by the grid point management unit 1124, and Figure 6(c) displays an image overlaid with the deformed grid and the anatomical structure of the analyzed region.

As shown in Figure 6(a), a square grid is set in the reference image. As a result of moving the centroid 'c' of the quadrilateral regions based on the movement of feature points 'f by the region change unit 1123, the grid management unit 1124 may deform the grid, as shown in Figure 6(b). Figure 6(b) can be regarded as information reflecting the temporal variation of the shape of the analysis target area. The image generation unit 113 may generate an anatomical image by superimposing the grid on at least one image showing the anatomy of the analysis target area (i.e., the second three-dimensional time series image). Thus, the image generation unit 113 may generate an anatomical image by superimposing the grid on at least the second three-dimensional time series image with the deformed grid. This allows medical personnel to visually grasp the temporal variations of the analysis target area in the second three-dimensional time series images, which are image sets showing the anatomy of the subject P.

### <Image Acquisition Sequence>

In the information processing system S, according to the embodiment, both the first three-dimensional time series image and the second three-dimensional time series image may be generated by MRI imaging apparatus 2 imaging subject P. Therefore, it is not necessary to change imaging equipment to capture the first and second three-dimensional time series images of subject P. The first and second three-dimensional time series images can be sequentially captured, and from the perspective of subject P, subject P only needs to lie on the bed provided by the MRI imaging apparatus 2, and the capture of the first and second three-dimensional time series images can be completed. Additionally, when capturing the first and second three-dimensional time series images sequentially, either imaging sequence can be performed first.

The time difference between the capture times of the first three-dimensional time series image and the second three-dimensional time series image may fall within a predetermined time interval (e.g., within 10 minutes, 5 minutes, 3 minutes, or 1 minute), depending on the size of the analysis target area (and the number of imaging frames). That is, the image acquisition unit 110 may determine whether the time difference between the capture times of the first and second three-dimensional time series images falls within a predetermined time interval, thereby determining whether the first and second three-dimensional time series images were captured in the same imaging sequence.

The image acquisition unit 110 may acquire a set of images where the time difference between the capture times of the first three-dimensional time series image and the second three-dimensional time series image falls within a predetermined time interval, thereby obtaining a set of images captured in the same imaging sequence. Consequently, the image acquisition unit 110 can ensure that the capture times of images showing the anatomy of the analysis target area and images showing the blood flow distribution within the analysis target area fall within a predetermined time interval, thereby suppressing the deviation between the first and second three-dimensional time series images due to temporal changes in the imaging position.

Furthermore, the information processing device 1 may calculate indices related to blood flow by introducing anatomical knowledge of the target area without increasing the contrast of the second three-dimensional time series images, i.e., without using contrast agents, by concurrently using the second three-dimensional time series images and the first three-dimensional time series images, which are images showing the anatomy of the analysis target area. Consequently, the image acquisition unit 110 may acquire non-contrast-enhanced second three-dimensional time series images without using contrast agents, thereby reducing the burden on the subject P due to the use of contrast agents.

### <Processing Flow of Information Processing Method Executed by Information Processing Device 1>

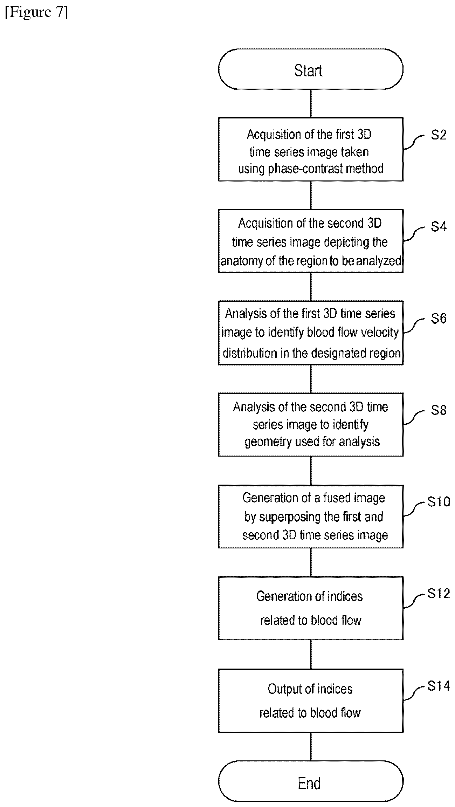

Figure 7 is an example flowchart explaining the flow of information processing executed by the information processing device 1 according to the embodiment. The processing in this flowchart may start, for example, when the information processing device 1 is booted.

The image acquisition unit 110 may acquire the first three-dimensional time series images, including the analysis target area of the subject p imaged using the phase-contrast method by the MRI imaging apparatus 2 (S2). Additionally, the image acquisition unit 110 may acquire the second three-dimensional time series images showing the anatomy of the subject p (S4).

The blood flow distribution identification unit 111 may identify the blood flow velocity distribution of the analysis target area by analyzing the first three-dimensional time series images using known blood flow visualization techniques (S6). The shape identification unit 112 may identify the shape of the analysis target area by analyzing the second three-dimensional time series image (S8).

The image generation unit 113 may generate a fused image by using known medical image synthesis techniques to overlay the first three-dimensional time series images and the second three-dimensional time series images (S10). The index generation unit 114 may generate indices related to the blood flow within the shape based on the blood flow velocity distribution and the shape in the fused image (S12).

The output unit 115 may output the shape of the analysis target area and indices related to blood flow, corresponding to the display unit of the information processing device 1 or a terminal capable of communicating with the information processing device 1 (S14). Specifically, the output unit 115 may output the shape of the analysis target area and indices related to blood flow in correspondence. Upon outputting the indices, the processing in this flowchart may end.

The present invention can be implemented as an information processing system that performs the functions described for the information processing device 1 according to the embodiment, as an information processing method executed by the information processing device, as a computer-readable recording medium storing a program for causing a computer to perform the functions, and as a program for causing a computer to perform the functions. These embodiments have effects similar to those described for the information processing device 1 according to the embodiment.

An embodiment of an information processing system that performs the functions described for the information processing device 1 according to the embodiment is further elaborated below. This information processing system may be as described earlier as the second aspect of the present invention. That is, this information processing system may include a blood flow distribution identification means that analyzes the first three-dimensional time series images containing the analysis target area of a subject imaged using phase-contrast imaging by an MRI (Magnetic Resonance Imaging) imaging device to identify the blood flow velocity distribution of the analysis target area, a shape identification means that analyzes images, which were acquired using a method different from phase-contrast imaging and show the anatomy of the subject, to identify the shape of the analysis target area, an image generation means that generates a fused image by overlaying the first three-dimensional time series images and the second three-dimensional time series images, an index generation means that generates indices related to blood flow within the shape based on the blood flow velocity distribution and the shape in the fused image, and an output means that outputs the shape and indices associated with the shape.

The information processing system described herein may correspond to the information processing device 1 in the aforementioned information processing system S. The blood flow distribution identification means may correspond to the blood flow distribution identification unit 111 described earlier. The shape identification means may correspond to the shape identification unit 112 described earlier. The image generation means may correspond to the image generation unit 113 described earlier. The index generation means may correspond to the index generation unit 114 described earlier. The output means may correspond to the output unit 115 described earlier.

### <Effects of the Information Processing Device 1 According to the Embodiment>

As described above, the information processing device 1 according to the embodiment can reduce the burden on the subject in blood flow measurements.

While the present invention has been described using embodiments, the technical scope of the present invention is not limited to the embodiments described above, and various modifications and changes are possible within the scope of the gist thereof. For example, all or part of the apparatus may be functionally or physically distributed or integrated in any unit. Moreover, new embodiments resulting from combinations of multiple embodiments are also included in the embodiments of the present invention. The effects of the new embodiments resulting from combinations encompass the effects of the original embodiments. Furthermore, the content of all publications, including patents and patent applications, referred to in this specification should be considered as being incorporated herein by reference, as if fully set forth herein.

### Explanation of symbols

1... Information processing device
10... Memory unit
11... Control unit
110... Image acquisition unit
111... Blood flow distribution identification unit
112... Shape identification unit
1120... Region segmentation unit
1121... Feature point extraction unit
1122... Tracking unit
1123... Region modification unit
1124... Grid point management unit
113... Image generation unit
114... Index generation unit
115... Output unit
2... MRI imaging device
S... Information processing system

## Claims

1. An information processing device comprising:
a blood flow distribution identification unit that analyzes a first three-dimensional time-series image, including an analysis target site of a subject imaged using phase-contrast imaging by an MRI (Magnetic Resonance Imaging) imaging device, and identifies a blood flow velocity distribution of the analysis target site;
a shape identification unit that analyzes a second three-dimensional time-series image indicating the anatomy of the subject imaged using a method different from phase-contrast imaging and identifies the shape of the analysis target site;
an image generation unit that generates a fused image by overlapping the first three-dimensional time-series image and the second three-dimensional time-series image;
an index generation unit that generates an index related to blood flow flowing inside the shape based on the blood flow velocity distribution and the shape in the fused image;
and an output unit that associates the shape with the index and outputs them.

2. The information processing device of Claim 1, wherein the analysis target site is the heart and large blood vessels of the heart, and
wherein the shape identification unit comprises:
a region segmentation unit that divides a reference image, which is one of multiple temporal images constituting the second three-dimensional time-series image, into multiple regions, including the region of the heart;
a feature point extraction unit that extracts one or more feature points in the reference image;
a tracking unit that tracks the transition of the feature points in the second three-dimensional time-series image, starting from the feature points; and
a region modification unit that temporally changes the shape of the multiple regions based on the tracking results of the feature points.

3. The information processing device of Claim 2, wherein the index generation unit generates an index related to blood flow flowing inside the region of the heart identified by the shape identification unit.

4. The information processing device of Claim 2 or 3, wherein the shape identification unit further comprises a grid point management unit that generates a grid in the reference image and deforms the grid based on the tracking results of the feature points, and
wherein the image generation unit generates at least an anatomical image overlaid with the deformed grid in the second three-dimensional time-series image.

5. The information processing device of any one of Claims 1 to 4, wherein the information processing device further comprises an image acquisition unit that acquires a set of images where the difference between the imaging time of the first three-dimensional time-series image and the imaging time of the second three-dimensional time-series image is within a predetermined time.

6. The information processing device of Claim 5, wherein the predetermined time is within 10 minutes.

7. The information processing device of Claim 5 or 6, wherein the image acquisition unit acquires non-contrast second three-dimensional time-series images imaged without using contrast agents.

8. The information processing device of any one of Claims 1 to 7, wherein the method different from the phase-contrast imaging is selected from the group consisting of SSFP (Steady-State Free Precession), Fast Gradient Echo imaging, Gradient Echo imaging, and Black Blood imaging.

9. The information processing device of any one of Claims 1 to 8, wherein the index related to blood flow is selected from the group consisting of left ventricular volume, ejection fraction, wall motion abnormality, stroke volume, presence of accelerated blood flow, quantification of regurgitation volume at cardiac valves, quantification of intracardiac shunt rate, quantification of local blood flow, flow through valves such as aortic valve, mitral valve, pulmonary valve, and tricuspid valve, shear stress on the vascular wall (Wall Shear Stress; WSS), index value of WSS temporal variation, flow or energy loss in the aorta, deformation amount of blood vessels or heart, longitudinal expansion and contraction of blood vessels or heart, change in curvature or tortuosity of the aortic arch, change in ventricular volume, and change in ejection fraction.

10. The information processing device of Claim 1, wherein the information processing device comprises:
a blood flow distribution identification unit that analyzes a first three-dimensional time-series image including an analysis target site of a subject imaged using phase-contrast imaging by an MRI (Magnetic Resonance Imaging) imaging device, and identifies a blood flow velocity distribution of the analysis target site;
a shape identification unit that analyzes a second three-dimensional time-series image indicating the anatomy of the subject imaged using a method different from phase-contrast imaging, and identifies the shape of the analysis target site;
an image generation unit that generates a fused image by overlapping the first three-dimensional time-series image and the second three-dimensional time-series image;
an index generation unit that generates an index related to blood flow flowing inside the shape based on the blood flow velocity distribution and the shape in the fused image;
and an output unit that associates the shape with the index and outputs them,
wherein the analysis target site is the heart and large blood vessels of the heart,
wherein the method different from the phase-contrast imaging is selected from the group consisting of SSFP, Fast Gradient Echo imaging, Gradient Echo imaging, and Black Blood imaging,
wherein the index related to blood flow is selected from the group consisting of left ventricular volume, ejection fraction, wall motion abnormality, stroke volume, presence of accelerated blood flow, quantification of regurgitation volume at cardiac valves, quantification of intracardiac shunt rate, quantification of local blood flow, flow through valves such as aortic valve, mitral valve, pulmonary valve, and tricuspid valve, shear stress on the vascular wall (Wall Shear Stress; WSS), index value of WSS temporal variation, flow or energy loss in the aorta, deformation amount of blood vessels or heart, longitudinal expansion and contraction of blood vessels or heart, change in curvature or tortuosity of the aortic arch, change in ventricular volume, and change in ejection fraction,
wherein the shape identification unit comprises:
a region segmentation unit that divides a reference image, which is one of multiple temporal images constituting the second three-dimensional time-series image, into multiple regions including the region of the heart;
a feature point extraction unit that extracts one or more feature points in the reference image;
a tracking unit that tracks the transition of the feature points in the second three-dimensional time-series image starting from the feature points;
and a region modification unit that temporally changes the shape of the multiple regions based on the tracking results of the feature points,
wherein the index generation unit generates an index related to blood flow flowing inside the region of the heart identified by the shape identification unit,
wherein the shape identification unit further comprises a grid point management unit that generates a grid in the reference image and deforms the grid based on the tracking results of the feature points,
wherein the image generation unit generates at least an anatomical image overlaid with the deformed grid in the second three-dimensional time-series image,
wherein the information processing device further comprises an image acquisition unit that acquires a set of images where the difference between the imaging time of the first three-dimensional time-series image and the imaging time of the second three-dimensional time-series image is within 10 minutes, and
wherein the image acquisition unit acquires non-contrast second three-dimensional time-series images imaged without using contrast agents.

11. An information processing system, comprising:
a blood flow distribution determination means for analyzing a first three-dimensional time-series image including an analysis target site of a subject imaged using phase-contrast imaging by an MRI (Magnetic Resonance Imaging) imaging device and identifying a blood flow velocity distribution of the analysis target site;
a shape identification means for analyzing a second three-dimensional time-series image indicating the anatomy of the subject imaged using a method different from phase-contrast imaging and identifying the shape of the analysis target site;
an image generation means for generating a fused image by superimposing the first three-dimensional time-series image and the second three-dimensional time-series image;
an index generation means for generating an index related to blood flow flowing inside the shape based on the blood flow velocity distribution and the shape in the fused image; and
an output means for associating the shape with the index and outputting them.

12. A method for information processing, wherein a processor executes:
a step of analyzing a first three-dimensional time-series image including an analysis target site of a subject imaged using phase-contrast imaging by an MRI imaging device to identify a blood flow velocity distribution of the analysis target site;
a step of analyzing a second three-dimensional time-series image indicating the anatomy of the subject imaged using a method different from phase-contrast imaging to identify the shape of the analysis target site;
a step of generating a fused image by superimposing the first three-dimensional time-series image and the second three-dimensional time-series image;
a step of generating an index related to blood flow flowing inside the shape based on the blood flow velocity distribution and the shape in the fused image; and
a step of associating the shape with the index and outputting them.

13. A computer-readable recording medium storing a program that enables a computer to realize:
a function for analyzing a first three-dimensional time-series image including an analysis target site of a subject imaged using phase-contrast imaging by an MRI imaging device to identify a blood flow velocity distribution of the analysis target site;
a function for analyzing a second three-dimensional time-series image indicating the anatomy of the subject imaged using a method different from phase-contrast imaging to identify the shape of the analysis target site;
a function for generating a fused image by superimposing the first three-dimensional time-series image and the second three-dimensional time-series image;
a function for generating an index related to blood flow flowing inside the shape based on the blood flow velocity distribution and the shape in the fused image; and
a function for associating the shape with the index and outputting them.

14. A program for enabling a computer to realize:
a function for analyzing a first three-dimensional time-series image including an analysis target site of a subject imaged using phase-contrast imaging by an MRI imaging device to identify a blood flow velocity distribution of the analysis target site;
a function for analyzing a second three-dimensional time-series image indicating the anatomy of the subject imaged using a method different from phase-contrast imaging to identify the shape of the analysis target site;
a function for generating a fused image by superimposing the first three-dimensional time-series image and the second three-dimensional time-series image;
a function for generating an index related to blood flow flowing inside the shape based on the blood flow velocity distribution and the shape in the fused image; and
a function for associating the shape with the index and outputting them.
